# EUROPEAN PATENT SPECIFICATION

(11) **EP 1 366 151 B1**
(45) Date of publication and mention of the grant of the patent: **24.10.2007**
(21) Application number: 01964371.7
(22) Date of filing: 23.08.2001
(51) Int. Cl.: C12N 15/00

(54) **PRNA CHIMERA**
pRNA-CHIMÄRE
CHIMERE D'ARNP

(30) Priority: 23.08.2000 US 227393 P
(43) Date of publication of application: 03.12.2003
(73) Proprietor: Purdue Research Foundation, West Lafayette, IN 47906 (US)
(72) Inventor: GUO, Peixuan, West Lafayette, IN 47906 (US); HOEPRICH, Stephen, M., North Canton, OH 44720-6040 (US); SHU, Dan, West Lafayette, IN 47907 (US)
(74) Representative: Stolzenburg, Friederike
(86) International application number: PCT/US2001/026333
(87) International publication number: WO 2002/016596

(56) References cited:
- WO-A-93/24133
- WO-A-99/51755
- ZHANG C. ET AL.: "Use of circular permutation to assess six bulges and four loops of DNA-packaging pRNA of bacteriophage phi29" RNA, vol. 3, no. 3, March 1997 (1997-03), pages 315-323, XP008013652 cited in the application
- ZHANG C. ET AL.: "Circularly permuted viral pRNA active and specific in the packaging of bacteriophage phi29 DNA" VIROLOGY, vol. 207, 1995, pages 442-451, XP002230513 cited in the application
- GARVER K. AND GUO P.: "Boundary of pRNA functional domains and minimum pRNA sequence requirement for specific connector binding and DNA packaging of phage phi29" RNA, vol. 3, no. 9, September 1997 (1997-09), pages 1068-1079, XP008013679
- PECENKOVÁ T. AND PACES V.: "Molecular phylogeny of phi29-like phages and their evolutionary relatedness to other protein-primed replicating phages and other phages hosted by Gram-positive bacteria" JOURNAL OF MOLECULAR EVOLUTION, vol. 48, 1999, pages 197-208, XP002230514
- TAIRA K. NISHIKAWA S.: "Construction of several kinds of ribozymes: their reactivities and utilities" GENE REGULATION. BIOLOGY OF ANTISENSE RNA AND DNA. ERICKSON R.P. AND IZANT J.G. EDS. NEW YORK, RAVEN PRESS LTD., US, 1992, pages 35-54, XP002002021
- WEINER J.A. ET AL.: "Gamma protocadherins are required for synaptic development in the spinal cord" PROCEEDINGS OF THE NATIONAL ACADEMY OF SCIENCE OF USA, vol. 102, no. 1, 4 January 2005 (2005-01-04), pages 8-14,

### SEQUENCE LISTING

<110> GUO, Peixuan
   HOEPRICH, Stephen M
   SHU, Dan
<120> pRNA CHIMERA
<130> P-00042.P1.us
<140> Unassigned
   <141> 2001-08-23
<150> US 60/227,393
   <151> 2000-08-23
<160> 17
<170> PatentIn version 3.1
<210> 1
   <211> 120
   <212> RNA
   <213> Bacteriophage phi29/PZA
<400> 1
   ucaaugguac gguacuucca uugucaugug uauguugggg auuaaacccu gauugaguuc 60
   agcccacaua cuuuguugau ugguugucaa ucauggcaaa agugcacgcu acuuugauaa 120
<210> 2
   <211> 121
   <212> RNA
   <213> Artificial Sequence
<220>
   <223> circularly permuted pRNA from bacteriophage phi29
<400> 2
   ggaaugguac gguacuucca uugucaugug uauguugggg auuaaacccu gauugaguuc 60
   agcccacaua cuuuguugau ugguugucaa ucauggcaaa agugcacgcu acuuuccuaa 120
   a 121
<210> 3
   <211> 167
   <212> RNA
   <213> Artificial Sequence
<220>
   <223> RNA chimera containing phi29 pRNA and hammerhead ribozyme
<400> 3
   guugauuggu ugucaaucau ggcaaaagug cacgcuacuu ugaaaaacaa auucuaaaac 60
   ugaugagucc gugaggacga aagcuguaac acaaaaucaa ugguacggua cuuccauugu 120
   cauguguaug uuggggauua aacccugauu gaguucagcc cacauac 167
<210> 4
   <211> 24
   <212> RNA
   <213> Artificial Sequence
<220>
   <223> U7snRNA substrate
<400> 4
   guguuacagc ucuuuuagaa uuug 24
<210> 5
   <211> 46
   <212> RNA
   <213> Artificial Sequence
<220>
   <223> anti-12-Lox ribozyme
<400> 5
   acgcgguugu accugaugag uccgugagga cgaaacccgg agaaga 46
<210> 6
   <211> 25
   <212> RNA
   <213> Artificial Sequence
<220>
   <223> substrate RNA
<400> 6
   ucuucuccgg gucguacaac cgcgu 25
<210> 7
   <211> 77
   <212> RNA
   <213> Artificial Sequence
<220>
   <223> pRNA chimera
<220>
   <221> misc_feature
   <222> (7)..(11)
   <223> any nucleotide
<220>
   <221> misc_feature
   <222> (26)..(26)
   <223> spacer region
<220>
   <221> misc_feature
   <222> (51)..(54)
   <223> any nucleotide
<400> 7
   guugaunnnn ngucaaucau ggcaanuugu cauguguaug uuggggauua nnnncugauu 60
   gaguucagcc cacauac 77
<210> 8
   <211> 17
   <212> DNA
   <213> Artificial Sequence
<220>
   <223> linking loop
<400> 8
   taatacgact cactata 17
<210> 9
   <211> 94
   <212> RNA
   <213> Artificial Sequence
<220>
   <223> U7 substrate
<400> 9
   gggaaagcuu auaguguuac agcucuuuua gaauuugucu agcagguuuu cugacuucgg 60
   ucggaaaacg ccuaacguug caugccugca gguc 94
<210> 10
   <211> 47
   <212> RNA
   <213> Artificial Sequence
<220>
   <223> Hammerhead ribozyme
<400> 10
   ggcaaauucu aaaacugaug aguccgugag gacgaaagcu guaacac 47
<210> 11
   <211> 116
   <212> RNA
   <213> Bacteriophage SF5'
<400> 11
   gugagacauc guauuuacua uaauguaugu gugucggguu guuuucggau ugaguuccgc 60
   cgacagcagg ggagcguaaa acacucuugc auuaaaaaua ugacugauuu cgcuac 116
<210> 12
   <211> 121
   <212> RNA
   <213> Bacteriophage B103
<400> 12
   gacaaggugc aacacuuccu auaguauggu gcgugauugg gguauauccu gauugaguuc 60
   agcccacacg ccacggggau ugauagcccu cuuacuaaaa gugauuguuu cuuugucgcu 120
   u 121
<210> 13
   <211> 0
   <212> RNA
   <213> (not used)
<400> 13
   000
<210> 14
   <211> 120
   <212>RNA
   <213> Bacteriophage M2/NF
<400> 14
   gacaaggugc aacacuuccu auaguauggc acaugauugg gguauauccu gauugaguuc 60
   agcccacaug ucacggggau ugauaacccu cuuacuaaaa gugauuguuu cuuuguccuu 120
<210> 15
   <211> 115
   <212> RNA
   <213> Bacteriophage GA1
<400> 15
   uaaggcauug agguuaaaau auauaggcug ugcaacgggc auccuacugu aaaaaguacc 60
   guugacaggu uguugcgugg uagcaauacu auauauacuc ucucucguuu uauaa 115
<210> 16
   <211> 124
   <212> RNA
   <213> Artificial Sequence
<220>
   <223> aptpRNA
<400> 16
<210> 17
   <211> 188
   <212> RNA
   <213> Artificial Sequence
<220>
   <223> pRzApRNA
<400> 17
   gcuaguucua gaguugauug guugucaauc auggcaaaag ugcacgcuac uuugcaaaac 60
   aaauucuuua cugaugaguc cgugaggacg aaacggguca aaagcaaugg uacgguacuu 120
   ccauugucau guguauguug gggauuaaac ccugauugag uucagcccac auacgguacc 180
   ucgacguc 188

## Description

### Background

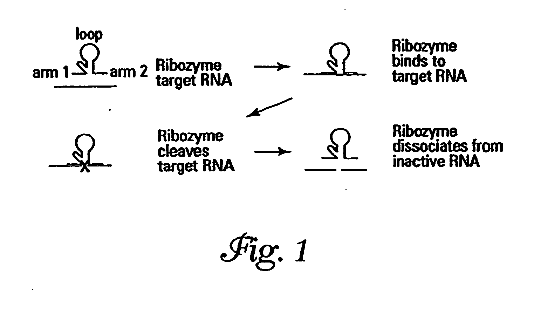

A ribozyme is an RNA enzyme capable of cleaving a target RNA molecule. Structurally, it is single-stranded RNA characterized by two "arms" positioned either side of a small loop. The ribozyme base pairs to a region on the target RNA that is complementary to the nucleotide sequence of its two arms. The loop region serves as an active catalytic center that performs the cleaving function on the target RNA (Fig. 1).

The use of ribozymes for treatment and prevention of diseases in plants, humans and animals has the potential to revolutionize biotechnology. Hammerhead ribozymes have, for example, been used to cleave RNA in transgenic plants and animals. However, despite numerous publications reporting the results of investigations in test tubes, reports on the successful use of hammerhead ribozymes in living organisms are relatively few (Perriman et al., Proc. Natl. Acad. Sci. USA 92:6175-6179 (1995)). Although it is clear that hammerhead ribozymes can cleave specific viral RNA or mRNA in test tubes, the efficiency of cleavage in cells is dramatically reduced due to instability and misfolding of the ribozyme in cells.

A major cause for the instability of ribozymes in an intracellular environment is degradation of the ribozyme by exonuclease present in the cells (Cotton et al., EMBO J. 8:3861-3866. (1989)). Exonucleases are enzymes that nonspecifically trim RNA from both ends. One method that has been used to block the intracellular degradation of ribozymes is to protect the ribozyme by connecting it atone end to a vector RNA, such as tRNA. However, due to refolding of the resulting chimera RNA, the ribozyme varied in efficiency compared to the unprotected ribozyme (Bertrand et al., RNA 3:75-88 (1997)). Tethering of a ribozyme to both ends of a tRNA has also been reported, but folding and/or activity was compromised (Vaish et al., Nucl. Acids Res. 26:523.7-5242 (1998)).

Taira and Nishikawa (Gene Regulation. Biology of Antisense RNA and DNA. Erickson RP and Izant JG, eds., Raven Press, Ltd., New York 1992, pp.35-54) also describe ribozyme molecules which are stabilized by being embedded into tRNAs. In Wo 99/515755, it is indicated that the higher order structure of the tRNA may protect the ribozyme from degradation by cellular nucleases.
Moreover, Zhang et al. (RNA 3 (1997), 315-323) report on a functional analysis of the bulge /loop structure of pRNA using circular permutation.

The potential to treat disease by using ribozymes to cleave RNA involved in cancer and pathogen infection is tremendous. The availability of a stabilized ribozyme that is resistant to degradation and is correctly folded such that it remains active in an intracellular environment would pave the way for the development of many important medical therapies.

### Summary of the Invention

The invention provides a circularly permuted chimeric pRNA molecule carrying a stabilized, properly folded, biologically active RNA. The pRNA chimera is formed from a circularly permuted pRNA region, and a spacer region that includes the biologically active RNA. The biologically active RNA is preferably a ribozyme or an antisense RNA. The spacer region is covalently linked at its 5' and 3' ends to the pRNA region. Optionally, the spacer region includes first and second nucleotide strings interposed between the biologically active moiety and the pRNA region.

The pRNA region has a compact stable secondary structure characteristic of bacteriophage pRNA sequences. Thus, in one embodiment of the pRNA chimera, the pRNA region includes a circularly permuted pRNA of a bacteriophage selected from the group consisting of φ29, SF5', B103, PZA, M2, NF and GA1. In another embodiment of the pRNA chimera, the pRNA region includes:
(i) in the 5' to 3' direction beginning at the covalent linkage of the pRNA with the 3' end of the spacer region
   a first loop 22;
   a second loop 24; and
   a lower stem-loop structure comprising a bulge,
   a first stem section 26 and a third loop 27;
(ii) a second stem section 20 interposed between the spacer region and the stem-loop structure;
(iii) a third stem section 21 interposed between the stem-loop structure and the first loop 22;
(iv) a fourth stem section 23 interposed between the first loop 22 and the second loop 24; and
(v) an opening defining 5' and 3' ends of the pRNA chimera, positioned anywhere within the pRNA region.

The invention also provides a method for making a pRNA chimera of the invention. A DNA encoding a pRNA chimera containing a pRNA region and a spacer region that includes a biologically active RNA is transcribed *in vitro* to yield the pRNA chimera. Optionally, the DNA encoding the pRNA chimera is generated using polymerase chain reaction on a DNA template, or the DNA is generated by cloning the DNA into a plasmid and replicating the plasmid.

The invention further provides a method for determining whether an RNA molecule interacts with a test molecule. A pRNA chimera that includes the RNA molecule of interest is immobilized on a substrate, then contacted with test molecule. Whether or not the test molecule interacts with the RNA of interest, such as by binding the RNA of interest, is then detected.

The invention also provides a DNA molecule that includes a nucleotide sequence that encodes a pRNA chimera containing a pRNA region and a spacer region that includes a biologically active RNA.

Also provided by the invention is a method for delivering a biologically active RNA to a cell, preferably a plant cell or an animal cell, such as human cell, in vitro. Alternatively, the invention provides a method for delivering a biologically active RNA to a non-animal cell. In one embodiment, a DNA molecule having a nucleotide sequence that operably encodes a pRNA chimera of the invention is introduced into the cell and transcribed to yield the biologically active RNA. In another embodiment, the pRNA chimera is directly transfected into the cell.

### Brief Description of the Figures

Figure 1 is a schematic depiction of target RNA cleavage by a representative ribozyme.
Figure 2 depicts the nucleotide sequence (SEQ ID NO:1) and secondary structure of wild-type φ29 (phi29) pRNA indicating the location and nomenclature of the loops and bulges (Zhang et al., RNA 3:315-323 (1997)).
Figure 3 depicts that nucleotide sequences of several pRNAs prior to circular permutation: (a) bacteriophage SF5' (SEQ ID NO:11), (b) bacteriophage B103 (SEQ ID NO:12), (c) bacteriophages φ29 and PZA (SEQ ID NO:1), (d) bacteriophage M2 and NF (SEQ ID NO:14), and (e) bacteriophage GA1 (SEQ ID NO:15) (Chen et al., RNA 5:805-818 (1999); and (f) aptpRNA (SEQ ID NO:16).
Figure 4 is a schematic depiction of various structural features of a pRNA chimera of the invention: (a) a whole pRNA chimera; (b) a spacer region component; (c) a pRNA region component.
Figure 5 is a schematic depiction of (a) the design of one embodiment of the pRNA chimera of the invention; and (b) exemplary circularly permuted pRNA (cpRNA) molecules showing various locations for the circle openings.
Figure 6 is a schematic depiction a possible mechanism of pRNA-ribozyme cleavage activity.
Figure 7 depicts (a) construction of tandem DNA templates for the synthesis of various cpRNAs (Zhang et al., Virology 207:442-451 (1995)); and (b) generalized circularly permuted pRNA structure (SEQ ID NO:2) with arrows indicating various new openings (Zhang et al., RNA 3:315-323 (1997)).
Figure 8 depicts an RNA chimera (SEQ ID NO:3) bound to a portion of the U7snRNA substrate (SEQ ID NO:4).
Figure 9 shows a denaturing urea gel evidencing successful cleavage of the substrate U7snRNA into its expected 69mer and 25mer cleavage products by both the ribozyme (Rz) and the pRNA-ribozyme chimera (RNA-Rz), using different concentrations of reactants for (a) and (b) as described in the text.
Figure 10 shows a denaturing urea gel evidencing successful cleavage of the substrate HBV-polyA into its expected 67mer and 70mer cleavage products, running as a single band, by the pRNA-ribozyme chimera RzApRNA
Figure 11 depicts an anti-12-LOX ribozyme (SEQ ID NO:5) bound to substrate RNA (SEQ ID NO:6).

### Detailed Description

Bacteriophage φ29 (phi29) is a double-stranded DNA virus. In 1987, one of the inventors, Dr. Peixuan Guo, discovered a viral-encoded 120 base RNA that plays a key role in bacteriophage φ29 DNA packaging (Guo et al. Science 236:690-694 (1987)). This RNA is termed packaging RNA or "pRNA". It binds to viral procapsids at the portal vertex (the site where DNA enters the procapsid) (Guo et al., Nucl. Acids Res. 15:7081-7090 (1987)) and is not present in the mature φ29 virion.

The pRNA contains two functional domains: one for procapsid binding and the other for a yet to be defined role in DNA translocation (Trottier et al., J. Virol. 71: 487-494 (1997); Trottier et al., J. Virol. 70: 55-61 (1996); Zhang et al., Virology 201:77-85 (1994)). Six copies ofpRNA are needed to package one genomic DNA (Trottier et al., J. Virol. 70:55-61 (1996); Trottier et al., J. Virol. 71, 487-494 (1997); Guo et al., Mol. Cell. 2, 149-155 (1998)). DNA packaging is completely blocked when one of the six slots is occupied by one inactive pRNA with a mutation at the 5' or 3' end (Trottier et al., J. Virol. 70:55-61 (1996); Trottier et al., J. Virol. 71:487-494 (1997)). Bacteriophage φ29 pRNA is associated with procapsids during the DNA translocation process (Chen et al., J. Virol. 71:3864-3871 (1997)). Inhibition data also suggests that the pRNA plays an essential role in DNA translocation (Trottier et al., J. Virol. 71:487-494 (1997)); Trottier et al. J. Virol. 70:55-6 (1996)). A Mg²⁺-induced conformational change of pRNA leads to its binding to the portal vertex (Chen et al. J. Virol. 71, 495-500 (1997)). The tertiary structure of the pRNA monomer and dimer has also reported (Zhang et al., Virology 81:281-93 (2001); Trottier et al., RNA 6(9):1257-1266 (2000); Chen et al. J. Biol. Chem. 275(23): 17510-17516 (2000); Garver et al., J. Biol. Chem. 275(4): 2817-2824 (2000)).

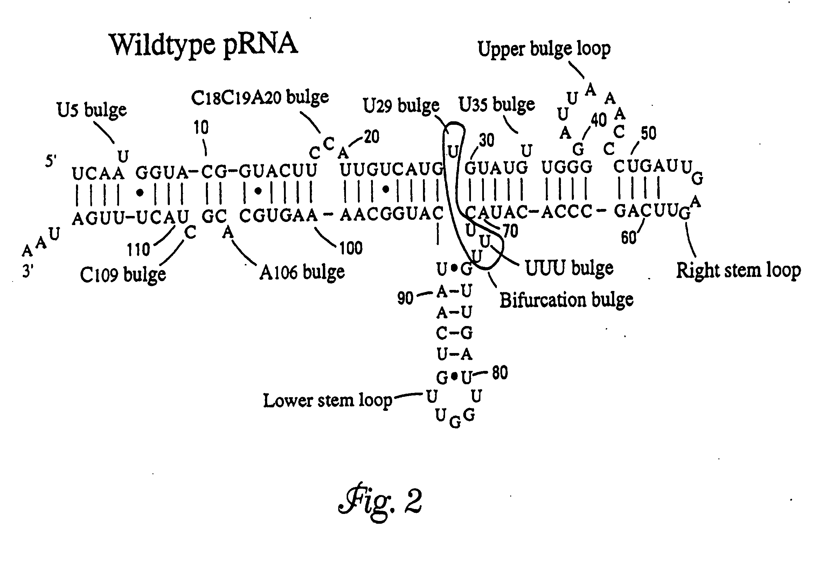

The nucleotide sequence (SEQ ID NO:1) of native full length φ29 pRNA (Guo et al., Nucl. Acids Res. 15:7081-7090 (1987)), as well as its predicted base-paired secondary structure, is shown in Fig. 2(a) (Zhang et al., RNA 3:315-323 (1997); Zhang et al., Virology 207:442-451 (1995)). The predicted secondary structure has been partially confirmed (Zhang et al., RNA 1:1041-1050 (1995); Reid et al., J. Biol. Chem. 269:18656-18661 (1994); Zhang et al., Virology 201:77-85 (1994); Chen et al., J. Virol. 71: 495-500 (1997)).

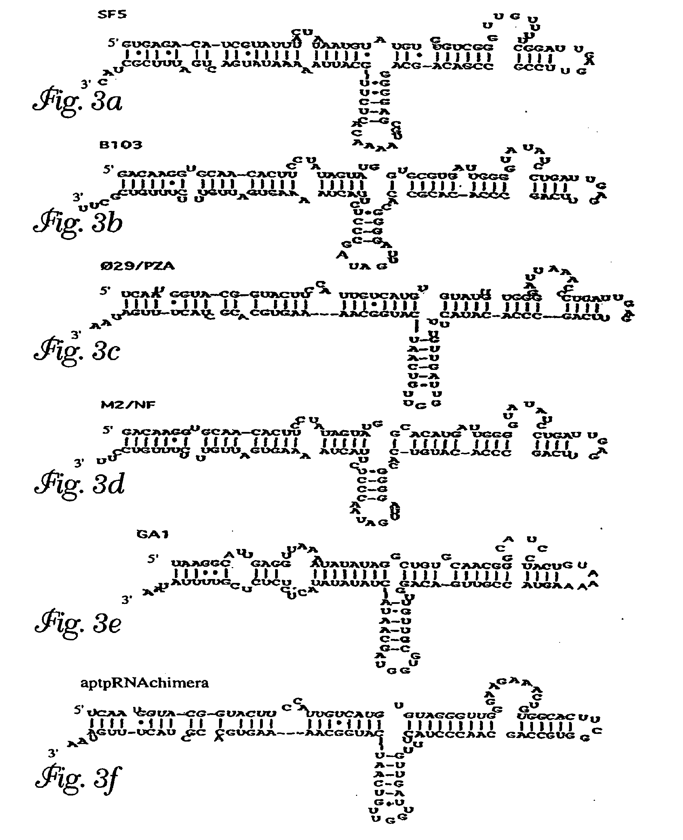

Phylogenetic analysis of pRNAs from phages SF5, B103, φ29, PZA, M2, NF and GA1 (Chen et al., RNA 5:805-818 (1999)) shows very low sequence identity and few conserved bases, yet the family of pRNAs appear to have strikingly similar and stable predicted secondary structures (3. 3). The pRNAs from bacteriophages SFS' (SEQ ID NO:11), B103 (SEQ ID NO:12), φ29/PZA (SEQ ID NO:1), M2/NF (SEQ ID NO:14), GA1 (SEQ ID NO:15) of *Bacillus subtilis* (Chen et al., RNA 5:805-818 (1999); and aptpRNA (SEQ ID N0:16) are all predicted to have a secondary structure that exhibits essentially the same structural features as shown in Fig. 2 for φ29 pRNA (Chen et al., RNA 5:805-818 (1999)). All have native 5' and 3' ends at the left end of a stem structure (as shown in Fig. 3) and contain the same structural features positioned at the same relative locations.

The pRNA of these bacteriophages, sharing as they do a single stable secondary structure, provide the framework for the pRNA chimera of the invention.

Secondary structure in an RNA molecule is formed by base pairing among ribonucleotides. RNA base pairs include G-C, A-T and U-G. Predictions of secondary structure are preferably made according to the method of Zuker and Jaeger, for example by using a program known by the trade designation RNASTRUCTURE 3.6, written by David H. Mathews (Mathews et al., J. Mol. Biol. 288:911-940 (1999); see also Zuker, Science 244:48-52 (1989); Jaeger et al., Proc. Natl. Acad. Sci. USA 86:7706-7710 (1989); Jaeger et al., Meth. Enzymol. 183:281-306 (1990)). This program is publicly available on the worldwide web at the homepage of the laboratory of Douglas Turner at the University of Rochester at rna.chem.rochester.edu/RNAstructure.html and runs on MS Windows 95, 96, ME, 2000 and NT4. The program is also publicly available on the worldwide web at Michael Zuker's homepage at Rensselaer Polytechnic Institute (bioinfo.math.rpi.edu/~zukerm/home.html); his homepage offers online folding and a version of the algorithm that can be compiled on Silicon Graphics, Sun, or DEC Alpha workstations. The structure with the lowest energy (i.e., the optimal structure) is chosen.

Secondary structures of RNA can be characterized by stems, loops and bulges. A "stem" is a double-stranded section of two lengths of base-paired ribonucleotides. Stem sections contain at least 2 base pairs and are limited in size only by the length of the RNA molecule. A "loop" is a single-stranded section that typically includes at least 3 ribonucleotides and is also limited in size only by the length of the RNA molecule. In a "stem loop", the 5' and 3' ends of the loop coincide with the end of a base-paired stem section. In a "bulge loop", the loop emerges from along the length of a stem section. The 5' and 3' ends of a bulge loop are typically not base paired although they may potentially be (see, e.g., G40 and C48 of the bulge loop in the φ29 pRNA structure; Fig. 2). A "bulge" is an unpaired single stranded section of about 1 to about 6 ribonucleotides present along the length of (or between) stem sections. Note that there is no clear line between a large "bulge" and a small "bulge loop." Herein, where the term "bulge" is used, it also includes a small "bulge loop" (i.e., a bulge loop of less than about 7 ribonucleotides).

The secondary structure of an RNA molecule is determined by the nature and location of the base pairing options along its length. RNA secondary structure is degenerate; that is, different primary ribonucleotide sequences can yield the same base pairing configurations and hence the same secondary structure. In a way, it is akin to the way multiple amino acid sequences can produce the same secondary structure, for example an α-helix.

A single secondary structure is dictated by a number of different primary sequences in predictable and well-understood ways. For example, single or pairs of nucleotides can generally be added, removed, or substituted without altering the overall base pairing interactions within the RNA molecule and without interfering with its biological function. This is particularly true if one or a few base pairs of nucleotides are removed, added or substituted along double-stranded hybridized length of the molecule, or if one or more nucleotides is removed, added or substituted in the single-stranded loop regions. For example, although GC base pairs and AT base pairs differ slightly in their thermodynamic stability, one can generally be substituted for another at a site within the double-stranded length without altering the secondary structure of an RNA molecule. GC base pairs are preferred in the stem region due to their added stability. Changes in secondary structure as a result of addition, deletion or modification of nucleotides can be readily assessed by applying the secondary structure prediction algorithm of Zuker and Jaeger as described above. The pRNA region of the RNA chimera can accommodate substantial variation in primary sequence without an appreciable change in secondary structure.

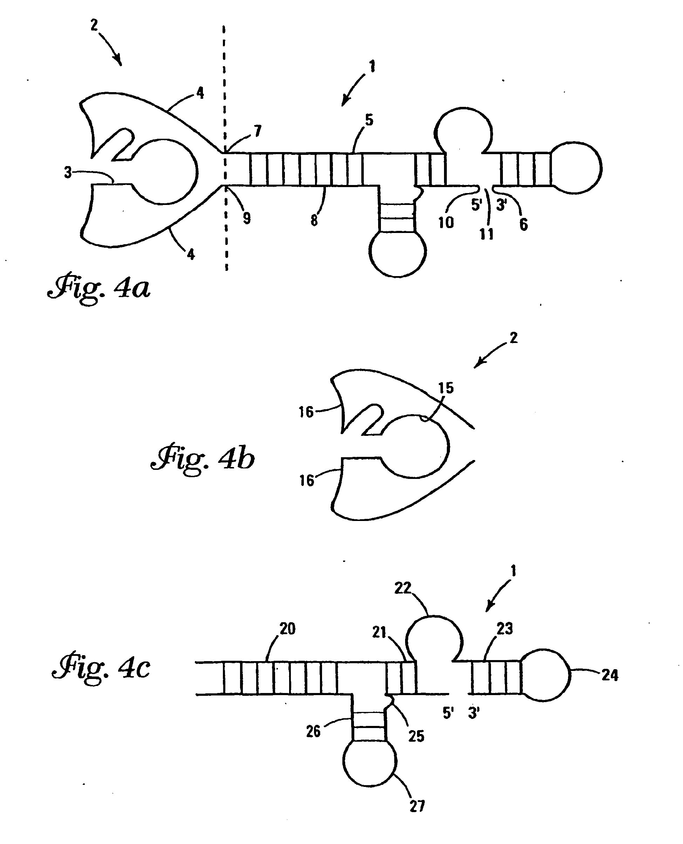

The pRNA chimera of the invention consists essentially of a pRNA region having the secondary structure exemplified in Fig. 3 (and schematically depicted in Fig. 4, as detailed below), interrupted by (i.e., flanking) a heterologous spacer region that contains a biologically active moiety, such as a ribozyme. The secondary structure of the pRNA region of the pRNA chimera is the common secondary structure that characterizes the pRNA from bacteriophages φ29, SF5', B103, PZA, M2, NF and GA1. The spacer region is termed "heterologous" because all or a portion of its nucleotide sequence is engineered or it is obtained from an organism other than the bacteriophage. It is the presence of the heterologous spacer region that renders the construct "chimeric" for the purposes of this invention. The pRNA chimera is useful as a vehicle to carry and deliver a ribozyme or other biologically active moiety to a target molecule or location. Since both ends of the ribozyme are connected to pRNA, the linkage is expected to protect the sensitive ribozyme from degradation and to assist the biologically active moiety to fold appropriately.

Notably, the ability of the pRNA chimera to perform its intended function of protecting and carrying a biologically active moiety depends not on the primary nucleotide sequence of the pRNA region (the primary structure), but on the secondary structure (base pairing interactions) that the pRNA region assumes as a result of its primary ribonucleotide sequence. The "pRNA region" of the pRNA chimera is so termed because it has a secondary structure, although not necessarily an RNA sequence, characteristic of a native bacteriophage pRNA molecule. Therefore, unless otherwise specified, the term "pRNA region" as used herein includes naturally occurring (native) pRNA sequences, nonnaturally occurring (nonnative) sequences, and combinations thereof provided that they yield the secondary structure characteristic of naturally occurring (native) bacteriophage pRNA as described herein. Stated another way, the term "pRNA region" is not intended to be limited to only those particular nucleotide sequences native to pRNA. The pRNA region can thus contain any nucleotide sequence which results in the secondary structure shown in Fig. 4. Nucleotide sequences that fold into the aforesaid secondary structure include naturally occurring sequences, those that are derived by modifying naturally occurring pRNA sequences, and those that are designed *de novo,* as well as combinations thereof. One of skill in the art can readily determine whether a nucleotide sequence will fold into the secondary structure shown in Fig. 4 and described herein by applying a secondary structure algorithm, such as RNASTRUCTURE as described above, to the nucleotide sequence.

Examples of nucleotide sequences that, when folded, yield the secondary structure of the pRNA region of the pRNA chimera of the invention are shown in Fig. 3. They include pRNA sequences from bacteriophages SF5' (SEQ ID NO:11), B103 (SEQ ID NO:12), φ29/PZA (SEQ ID NO:1), M2/NF (SEQ ID NO:14), GA1 (SEQ ID NO:15) as well as the aptpRNA (SEQ ID NO:16).

In embodiments of the pRNA chimera wherein the pRNA region includes or is derived from a naturally occurring pRNA, the spacer region of the pRNA chimera is covalently linked to the pRNA region at what can be considered the "native" 5' and 3' ends of a pRNA sequence, thereby joining the native ends of the pRNA region. The pRNA region of the pRNA chimera is optionally truncated when compared to the native bacteriophage pRNA; in those embodiments, and that as a result the "native" 5' and 3' ends of the pRNA region simply refer to the nucleotides that terminate or comprise the actual end of the truncated native pRNA. An opening is formed in the pRNA region to linearize the resulting pRNA chimera, effecting a "circular permutation" of the pRNA as detailed below. It should nonetheless be understood that the term "circularly permuted pRNA region" is not limited to naturally occurring pRNAs that have been circularly permuted but instead is intended to have the broader meaning of RNA having a pRNA-like secondary structure as shown in Fig. 4(c), including an opening in the pRNA region that forms the 5' and 3' ends of the pRNA chimera.

Examples of pRNA chimera of the invention are those formed from the pRNAs of bacteriophages SF5' (SEQ ID NO:11), B103 (SEQ ID NO:12), φ29/PZA (SEQ ID NO:1), M2/NF (SEQ ID NO:14), GA1 (SEQ ID NO:15) as well as aptpRNA (SEQ ID N0:16) by joining the native 5' and 3' ends to the spacer region and introducing an opening elsewhere in the pRNA region, as described herein. Another example of a pRNA chimera of the invention is: where N represents any nucleotide, without limitation and j is an integer between about 4 to about 8, preferably about 5. The spacer region is represented by Nₘ - B - Nₙ where Nₙ and Nₘ are nucleotide strings that are optionally included in the spacer region, and B includes the biologically active moiety. Preferably, B is a ribonucleotide sequence that includes a biologically active RNA. Both m and n can be independently zero or any integer. Preferably, m and n are independently at least about 3, more preferably at least about 5, and most preferably at least about 10. Further, n and m are independently preferably at most about 300, more preferably at most about 50, and most preferably at most about 30.

Further, since the pRNA region of the pRNA chimera is defined by its secondary structure, still other examples of a pRNA chimera can be readily made by "mixing and matching" nucleotide fragments from, for example, SEQ ID NO:s 1, 2, 7, 11, 12, 14, 15 and 16 that fold into particular secondary structural features (bulges, loops, stem-loops, etc.) provided that the resulting nucleotide sequence folds into the overall secondary structure as shown in Fig. 4. For example, nucleotides encoding bulge loop 22 from bacteriophage SF5' pRNA (SEQ ID NO:11) could be substituted for the nucleotides encoding bulge loop 22 in the φ29 pRNA (SEQ ID NO:1) to yield a pRNA region as described herein. Likewise, any number of artificial sequences can be substituted into SEQ ID NO:s 1, 2, 7, 11, 12, 14, 15 and 16 to replace nucleotide sequences that fold into one or more structural features (or portions thereof) to form a pRNA region as described herein. See, for example, aptpRNA (Fig. 3(f)) which was derived in that fashion from φ29 pRNA. The overarching principle is that the overall secondary structure of the pRNA region is the secondary structure common to the bacteriophage pRNAs, as schematically depicted in Fig. 4.

Importantly, the resulting pRNA chimera is not a circular molecule; rather, it is linearized due to a circular permutation of the pRNA region (Zhang et al., RNA 3:315-323 (1997); Zhang et al., Virology 207:442-451 (1995)). Briefly, an opening (i.e., a cleavage or break point) is provided in the pRNA region at any designated site to form the actual 5' and 3' ends of the RNA chimera. These 5' and 3' ends are at "nonnative" positions with respect to a naturally occurring linear pRNA.

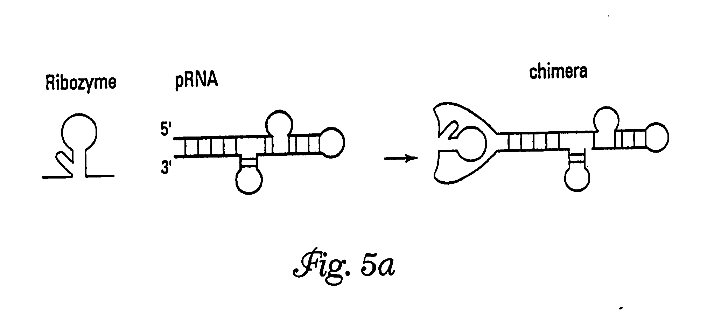

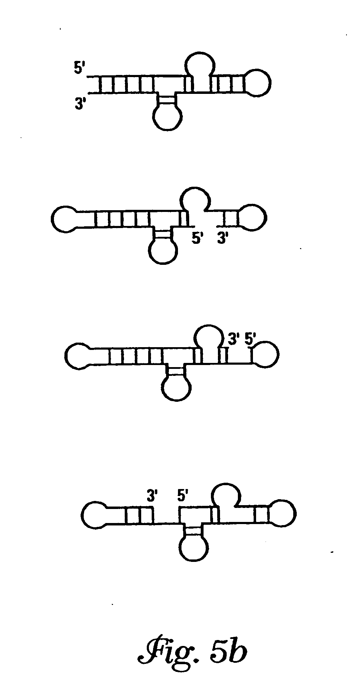

Fig. 5(a) shows how a pRNA chimera of the invention can be formed from a ribozyme and a pRNA region. The 5' and 3' ends of the pRNA can be engineered into any desired site on the circularly permuted pRNA chimera. Fig. 5(b) shows exemplary circularly permuted RNA molecules showing various locations for the circle openings.

Fig. 4 depicts various structural features that characterize a pRNA chimera of the invention. As shown in Fig. 4(a), the linear molecule includes a pRNA region 1 and a spacer region 2. Spacer region 2 contains a biologically active moiety 3, in this case a ribozyme; flanked by ribonucleotide strings 4. The pRNA region I is bifurcated; it includes a first pRNA segment 5 having 3' end 6 and "native" 5' end 7, and a second pRNA segment 8 having "native" 3' end 9 and 5' end 10. Ends 6 and 10 are the actual terminal ends of the pRNA chimera. Opening 11 renders the molecule linear and can be positioned anywhere in pRNA region I by the relocation of ends 6 and 10.

Spacer region 2 is shown in detail in Fig. 4(b). Ribozyme 3 is composed of a catalytic domain 15 flanked by target-binding sequences 16.

pRNA region 1 is shown in detail in Fig. 4(c). Overall, pRNA region 1 is characterized by a stem-loop secondary structure, wherein loop 24 is relatively small and the base-pairing in the stem (essentially stem sections 20, 21 and 23) is interrupted by structures on either side of loop 24. Bulge loop 22 is positioned 5' of loop 24. Positioned 3' of loop 24 is a stem-loop structure that contains bulge 25, stem 26 and loop 27.

Stem section 20 can be any number of ribonucleotides in length and can contain an unlimited number of bulges provided it is still able to base pair. Preferably, stem section 20 contains at least about 4, more preferably at least about 10 base pairs; further, it preferably it contains at most about 50, more preferably at most about 40 base pairs. Preferably stem section 20 contains about 0 to about 8 bulges; more preferably it contains about 0 to about 4 bulges.

Stem section 21 preferably contains 5-13 base pairs and 0-2 bulges.

Bulge loop 22 preferably contains 5-12 bases.

Stem section 23 preferably contains 3-12 base pairs and 0-2 bulges.

Loop 24 preferably contains 3-8 bases.

Bulge 25 preferably contains 0-5 bases.

Stem 26 preferably contains 4-8 base pairs and 0-2 bulges.

Loop 27 preferably contains 3-10 bases.

Tertiary interactions within an RNA molecule may result from nonlocal interactions of areas of the RNA molecule that are not near to each other in the primary sequence. Although native bacteriophage pRNA appears to exhibit tertiary interactions between bulge loop 22 and loop 27 (Chen et al., RNA 5:805-818 (1999); Guo et al, Mol. Cell. 2:149-155 (1998)) it should be understood that the pRNA chimera of the invention is not limited to RNA molecules exhibiting any particular tertiary interactions.

In one embodiment, the pRNA chimera of the invention contains at least 8, more preferably at least 15, most preferably at least 30 consecutive ribonucleotides found in native SF5' pRNA (Fig. 3(a)), B103 pRNA (Fig. 3(b)), φ29/ PZA pRNA (Fig. 3(c)), M2/NF pRNA (Fig. 3(d)), GA1 pRNA (Fig. 3(e)), or aptpRNA (Fig. 3(f)), preferably native φ29 pRNA. Most preferably, the pRNA region of the pRNA chimera contains at least a φ29 pRNA sequence that starts at ribonucleotide 23, preferably at ribonucleotide 20, and ends at ribonucleotide 95, preferably ribonucleotide 97, in the φ29 pRNA sequence (Fig. 2). In addition or in the alternative, the nucleotide sequence of the pRNA region of the pRNA chimera is preferably at least 60% identical to, more preferably 80% identical to, even more preferably 90% identical to, and most preferably 95% identical to the nucleotide sequence of a corresponding native SF5' pRNA (Fig. 3(a)), B103 pRNA (Fig. 3(b)), φ29/ PZA pRNA (Fig. 3(c)), M2/NF pRNA (Fig. 3(d)), GA1 pRNA (Fig. 3(e)), or the aptpRNA chimera (Fig. 3(f)), most preferably φ29 pRNA (particularly bases 20-97).

Percent identity is determined by aligning two polynucleotides to optimize the number of identical nucleotides along the lengths of their sequences; gaps in either or both sequences are permitted in making the alignment in order to optimize the number of shared nucleotides, although the nucleotides in each sequence must nonetheless remain in their proper order. For example, the two nucleotide sequences are readily compared using the Blastn program of the BLAST 2 search algorithm, as described by Tatusova et al. (FEMS Microbiol Lett 1999, 174247-250). Preferably, the default values for all BLAST 2 search parameters are used, including reward for match =1, penalty for mismatch = -2, open gap penalty = 5, extension gap penalty = 2, gap x_dropoff= 50, expect =10, wordsize = 11, and filter on.

The covalent linkages between the biologically active moiety and the pRNA region can be direct or indirect but preferably are indirect. In an indirect linkage, the spacer region includes additional string(s) of ribonucleotides at one or both ends of the biologically active moiety. These ribonucleotide strings, if present, contain preferably at least about 3 ribonucleotides; and preferably contain at most about 300, more preferably at most about 30 ribonucleotides. Compositionally, the strings can contain any desired ribonucleotides, however it is preferably that ribonucleotide compositions are selected so as to prevent the ribonucleotide strings on either side of the biological moiety from base pairing with each other or with other parts of the pRNA chimera.

Preferably the biological activity of the biologically active RNA is an enzymatic activity or binding activity or both; for example, the biologically active moiety may function as or encode a ribozyme or other catalytic moiety.

It should be understood that the terms "nucleotide," "oligonucleotide," and "polynucleotide" as used herein encompass DNA, RNA, or combinations thereof, unless otherwise indicated. Further, the terms DNA and RNA should be understood to include not only naturally occurring nucleic acids, but also sequences containing nucleotide analogs or modified nucleotides, such as those that have been chemically or enzymatically modified, for example DNA phosphorothioates, RNA phosphorothioates, and 2'-O-methyl ribonucleotides. A preferred biologically active RNA is a ribozyme such as a hammerhead ribozyme or a hairpin ribozyme. Antisense RNA and other bioactive RNAs are also preferred.

A ribozyme is generally characterized by:
arm 1 -active enzyme center - arm 2
where arm 1 and arm 2 are sequences complementary to the target substrate to be cleaved by the ribozyme, and the active enzyme center is the catalytic center that cleaves the target RNA. The "arms" of the ribozyme typically contain at least about 7 nucleotides, preferably at least about 12 nucleotides; and typically contain at most about 100 nucleotides, preferably at most about 30 nucleotides. The nucleotide sequence of the arms can be engineered to hybridize to the nucleotide sequence of any desired target nucleic acid.

Advantageously, incorporating a biologically active RNA, e.g., a ribozyme, into the pRNA chimera of the invention protects the ends of the ribozyme thereby rendering the it resistant to exonuclease degradation. Moreover, the secondary structure of pRNA is compact and very stable. A pRNA domain defined by nucleotides 30-91 of φ29 pRNA is especially stable. The compactness and stability of pRNA allows the pRNA region and the ribozyme to fold independently. Proper folding of the inserted RNA is facilitated, thereby preserving its biological activity. The stable structure of the carrier pRNA region is retained as well. A major obstacle in designing molecules to deliver ribozymes, i.e., misfolding of the ribozyme and carrier region as a result of interactions between them, has thus been overcome by utilizing the very stable pRNA molecule as the carrier. That the activity of the ribozyme is retained in the circularly permuted pRNA chimera is especially significant because it means that the new 5' and 3' ends of the pRNA chimera can be positioned so as to "bury" them in the folded pRNA structure, thereby further protecting the pRNA chimera from degradation. These features suggest great promise for the use of the pRNA chimera of the invention as a ribozyme delivery vehicle in medical and veterinary applications.

The pRNA chimera of the invention employs a "circular permutation" of a bacteriophage pRNA. A "circularly permuted" RNA molecule (cpRNA) is a linear RNA molecule in which the native 5' and 3' ends are covalently linked. The linkage can be direct, or it can be indirect by using a spacer region. Since a cpRNA molecule is linear, new nonnative 5' and 3' ends are created by forming an opening in the molecule (i.e., a discontinuity in the pRNA sequence) at a different location. The pRNA chimera of the invention is linear as a result of a nonnative opening in the bacteriophage pRNA framework at a designated site, which circularly permutes the bacteriophage framework and forms the actual 5' and 3' ends of the pRNA chimera. As already noted, the nonnative opening can be at any desired location in the pRNA region. Examples of selected locations in, for example in φ29 pRNA can be found in Zhang et al., RNA 3:315-323 (1997) and Zhang et al., Virology 207:442-451 (1995). See also Garver et al., J. Biol. Chem. 275:2817-2824 (2000); Chen et al., J. Virology 71:495-500 (1997); Trottier et al., RNA 6:1257-1266 (2000); and Zhang et al., Virology 281:281-293 (2001).

The pRNA chimera of the invention can be synthesized chemically or enzymatically using standard laboratory protocols. The pRNA region is preferably transcribed from a DNA template that encodes it, although if desired it can be synthesized chemically. If synthesized chemically, the pRNA region optionally contains nonnative nucleotides (e.g., derivatized or substituted nucleotides) and/or nonnative bonds analogous to the phosphodiester bonds that characterize naturally occurring nucleic acids.

Preferably the pRNA region is transcribed or synthesized as a single RNA molecule. In one embodiment of the method, the spacer region is chemically or enzymatically linked to the "native" ends of the pRNA region to form a circular chimeric molecule. The pRNA is then cleaved at a predetermined site to form the linear, circularly permuted pRNA chimera.

When the spacer region is RNA, another embodiment of the method includes transcribing the entire pRNA chimera from a single DNA template that encodes the entire chimeric molecule. In another embodiment of the method, the RNA spacer region is produced separately, either via transcription from its own template or by chemical synthesis, after which it is ligated to the pRNA region.

Also included in the invention is a DNA molecule that includes a nucleotide sequence that encodes the pRNA chimera of the invention. The spacer region of the encoded chimera is necessarily RNA in this aspect of the invention. The DNA molecule can be linear or circular. It can be double stranded or single stranded; if single stranded, its complement is included in the term "DNA molecule" as well. A DNA molecule for use in introducing a pRNA into a cell preferably contains regulatory elements such that the pRNA chimera is operably encoded. A pRNA chimera is "operably encoded" by a DNA molecule when the DNA molecule contains regulatory elements that allow the pRNA chimera to be produced by transcription of the DNA molecule inside the cell. Such regulatory elements include at least a promoter. Optionally, the DNA molecule includes additional regulatory motifs that promote transcription of the RNA chimera, such as, but not limited to, an enhancer. The DNA molecule can be introduced into the host cell using anionic or cationic lipid-mediated delivery or other standard transfection mechanisms including electroporation, adsorption, particle bombardment or microinjection, or through the use of a viral or retroviral vector.

It should be noted that the pRNA chimera can, if desired, be introduced into the host cell directly. For example, a product available under the trade designation TRANSMESSENGER TRANSFECTION REAGENT (available from Qiagen), which a lipid-based formulation that is used in conjunction with a specific RNA-condensing enhancer and an optimized buffer, can be used to transfect the pRNA chimera into eukaryotic cells.

Optionally, the DNA molecule can contain one or more features that allow it to integrate into the cell's genome. For example, it can be delivered in the form of a transposon, a retrotransposon, or an integrating vector; alternatively, it can contain sequences that are homologous to genomic sequences that allow it to integrate via homologous recombination. On the other hand, the DNA molecule can be designed to exist within a cell as nongenomic DNA, e.g., as a plasmid, cosmid, episome and the like.

Transcription from a DNA template encoding the entire chimeric RNA Molecule can occur *in vitro* or within a cell. The cell can be in cell culture, or in an organism (*in vivo*) such as a plant, then it is a non-animal cell, or in a cell explanted from an organism (*ex.vivo*).

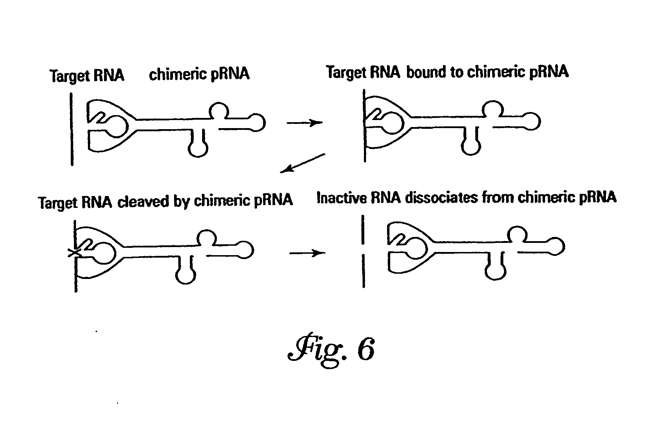

Advantageously, the pRNA chimera of the invention can be used to deliver a biologically active RNA molecule to a target within a cell. A DNA molecule having nucleotide sequence that operably encodes a circularly permuted pRNA region and a spacer region is introduced into a cell. The spacer region includes a biologically active RNA, and transcription of the DNA to yields the biologically active RNA. The biologically active molecule thus delivered is preferably a ribozyme, and the target is preferably viral or mRNA associated with a gene whose expression it is desirable to reduce. Fig. 6 shows a proposed mechanism for cleavage of a target RNA by a pRNA ribozyme chimera. An antisense RNA, which can target intracellular DNA or RNA, is also preferred as the biologically active molecule.

Surprisingly, conjugation of a ribozyme to a bifurcated pRNA region such that both ends of the ribozyme are covalently linked to the pRNA region does not render the ribozyme inactive, nor does it appear to interfere with the independent folding of the pRNA region or the ribozyme region. Because tethering of both ends of the ribozyme RNA is expected to also prevent degradation by exonuclease, the resulting pRNA-ribozyme chimera is expected to be useful to cleave undesired RNAs in plants and animals, including humans. Additionally, transgenic plants and animals with resistance to diseases can be developed by introducing DNA encoding the pRNA-ribozyme chimera into the genomic DNA of the cell.

The pRNA chimera of the invention is also useful *in vitro,* for example, for the characterization of RNA molecules. RNA molecules, particularly small RNA molecules, can be stabilized or "chaperoned" by inclusion in the spacer region of a pRNA chimera of the invention, which insures that they remain properly folded, active and exposed. For example, pRNA chimera containing an RNA of interest can be immobilized, covalently or noncovalently, on a substrate, such that the RNA of interest is presented. The immobilized pRNA chimera can then be contacted with test molecules, such as cellular extracts or components, to identify the constituents to which the RNA of interest binds or otherwise interacts. This is preferable to immobilizing the RNA of interest directly on the substrate, because direct immobilization can interfere with the folding of the RNA of interest and also block portions of the structure from contact with the test molecules. The pRNA chimera can also be used to stabilize RNAs in solution for use in binding assays, cleavage assays, diagnostics and the like.

### EXAMPLES

The present invention is illustrated by the following examples. It is to be understood that the particular examples, materials, amounts, and procedures are to be interpreted broadly in accordance with the scope and spirit of the invention as set forth herein.

### Example 1. Circularly Peemuted φ29 pRNA

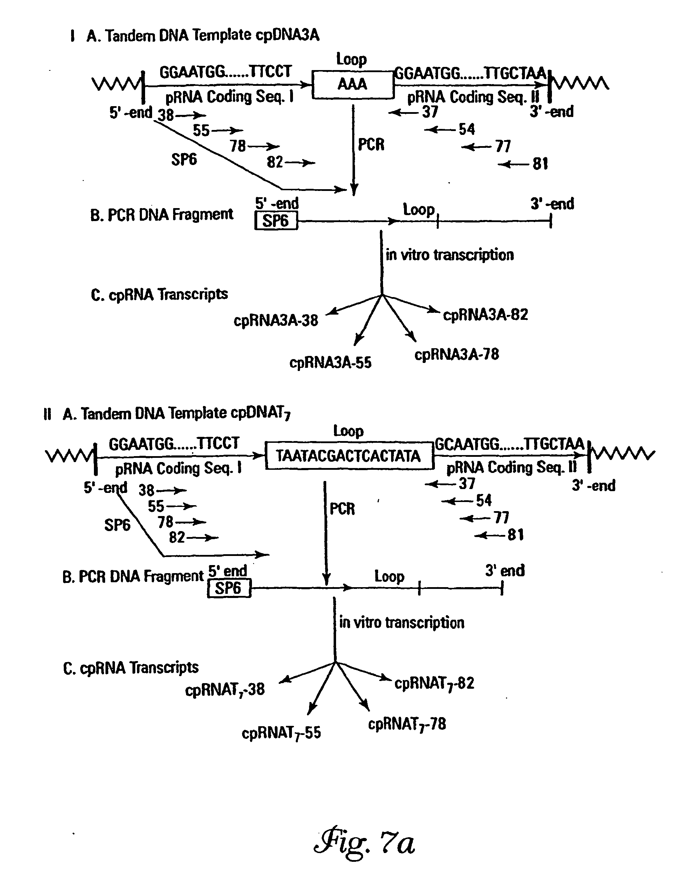

Circularly permuted pRNA (cpRNA) from bacteriophage φ29 was synthesized by way of transcription from a DNA template. Fig. 7(a) depicts construction of tandem DNA templates for the synthesis of various cpRNAs (Zhang et al., Virology 207:442-451 (1995)). Plasmids cpDNA3A (I) and cpDNAT7 (II) containing a tandem pRNA coding sequence were connected by 3- or 17-nucleotide synthetic loops, respectively. Four forward primers P38, P55, P78 and P82, which consisted of a SP₆ promoter (17-nucleotide) followed by residues 38-54, 55-71, 78-94 and 82-98 of the pRNA gene, and four reverse primers complementary to residues 37-21, 54-38, 77-59 and 81-65 of the pRNA gene were used to generate PCR fragments. The PCR DNA fragments were directly used as templates for *in vitro* transcription with SP₆ RNA polymerase. Four cpRNAs, cpRNA3A-38, cpRNA3A-55, cpRNA3A-78 and cpRNA3A-82 were generated from the DNA template cpDNA3A. Four other cpRNAs, cpRNAT₇-38, cpRNAT₇-55, cpRNAT₇-78 and cpRNAT₇-82 were synthesized from the DNA template cpDNAT₇. The resulting linear cpRNA transcript linked the native 5'-end of pRNA with its 3' end by way of small loop: AAA in the case of DNA template cpDNA3A and TAATACGACTCACTATA (SEQ ID NO:8) in the case of DNA template cpDNAT₇.

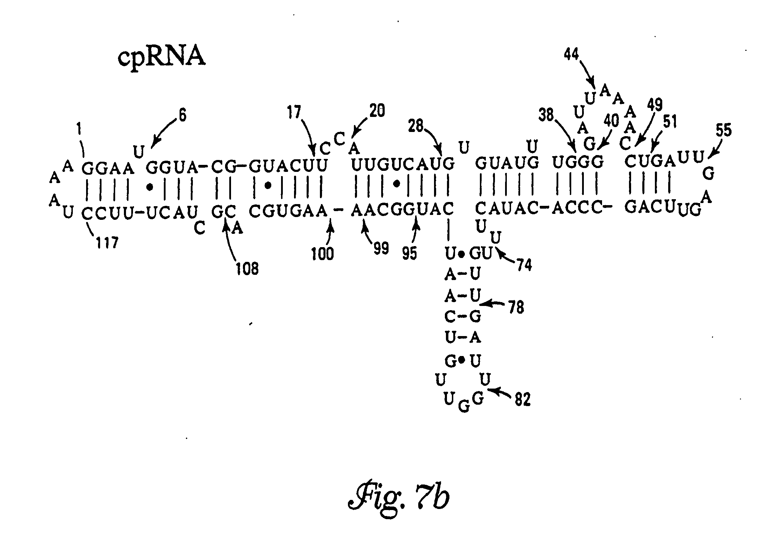

Other cpRNAs evaluated were joined by a AAAU loop. Fig. 7(b) shows generalized circularly permuted pRNA structure (SEQ ID NO:2) with arrows indicating various new openings (Zhang et al., RNA 3:315-323 (1997)). Wild-type sequences of 5'U1C2 and 3'A117G116 were changed to G1G2 and C 116C117, respectively, relative to wild-type pRNA.

To our surprise we found that insertion of sequences to link the native 5' and 3' ends of the pRNA molecule and relocation of the 5' and 3' ends somewhere else on the molecule does not interfere with the pRNA activity, since the cpRNA was still able to catalyze φ29 assembly.

### Example 2. In Vitro Activity of pRNA-Ribozyme Chimera

The loop used to connect the native termini of the pRNA in Example 1 did not itself possess any biological activity. However, we wondered whether an RNA sequence with biological activity would retain its activity if tethered at both ends to pRNA. It was decided to test a hammerhead ribozyme as the loop sequence.

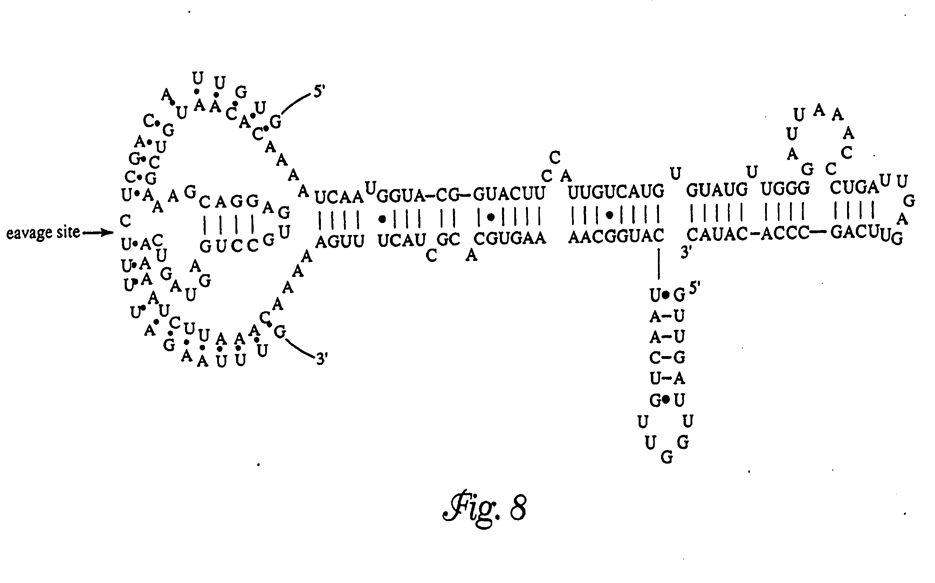

An *in vitro* model system (Fig. 8) as previously described in Cotton et al. (EMBO J. 8:3861-3866 (1989)) was modified and used as a control to test the functionality of a pRNA-ribozyme chimera. U7snRNA (SEQ ID NO:4) was selected as the target RNA. A chimeric RNA molecule, pRNA-Rz (SEQ ID NO:3), was synthesized.

The RNAs used in these experiments were generated by T7 polymerase *in vitro* transcription either using PCR or by cloning into a plasmid. The transcription products are as follows:

### T7 transcription of pRNA-Rz yields the 167mer:

### T7 transcription of U7 template yields the 94mer:

### T7 transcription of Rz template yields the 47mer:

5'GGCAAAUUCUAAAACUGAUGAGUCCGUGAGGACGAAAGCUGUA ACAC3' (SEQ ID NO:10).

The abilities of Rz (47 bases) (SEQ ID NO:10) and pRNA-Rz (167 bases) (SEQ ID NO:3) to cleave U7snRNA (SEQ ID NO:9) were compared. The Rz cleavage reaction was done as a control experiment to demonstrate that ribozyme reactions work correctly without any modifications. The cleavage reaction using pRNA-Rz was done to confirm that pRNA could be successfully used as a carrier molecule for ribozymes.

The cleavage reactions were carried out at 37°C for 90 minutes in the presence of 20 mM Tris pH 7.5, 150 mM NaCl, and 20 mMMgCl₂. Control reactions were done by substituting water for the RNA omitted. For example, the Rz control has no U7snRNA, but has water to make up the volume.

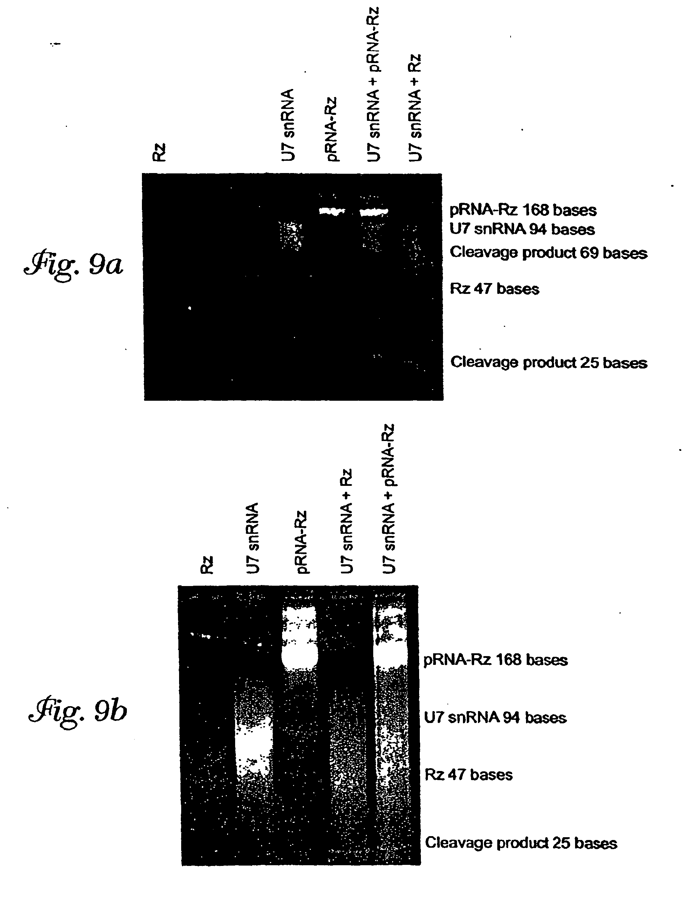

For the reaction shown in Fig. 9(a), the cleavage reactions used 10 pmol of Rz or pRNA-Rz to cleave 15 pmol U7snRNA. The samples were then run on a 16% Page / 8M urea denaturing gel in TBE. The gel was stained with ethidium bromide and visualized using EAGLE EYE II by Stratagene. Fig. 9(a) shows the successful results of the cleavage reaction. The predicted 69mer and 25mer cleavage products can be seen.

For the reaction shown in Fig. 9(b), the cleavage reactions used 48.58 pmol of Rz or pRNA-Rz to cleave 97.16 pmol U7snRNA. The samples were then run on a 15% Page / 8M urea denaturing gel in TBE. The gel was stained with ethidium bromide and visualized using EAGLE EYE II by Stratagene. The predicted 25-base cleavage product can be seen in Fig. 9(b). The predicted 69 base is hidden by the uncut U7snRNA.

This experiment confirmed successfully using pRNA as a carrier molecule for ribozymes. The finding that the hammerhead ribozyme retains activity in the pRNA-Rz construct has important implications. Independent folding of pRNA apparently and advantageously allows the ribozyme to fold into the correct structure and perform its function in cleaving target RNA. Furthermore, since both ends of the ribozyme are connected to pRNA, the linkage is expected to protect the ribozyme from exonuclease digestion in the cell. Thus, the ribozyme will be stable after expression in the transgenic plants or animals, solving a persistent problem that has stood in the way of therapeutic use of ribozymes.

### Example 3. In Vitro Activity of pRNA-Ribozyme Chimera against Hepatitis B Virus

Hepatitis is a serious disease that is prevalent in many countries worldwide. Hepatitis B virus (HBV) is one causative agent of this disease. HBV is an RNA virus. The RNA genome of HBV was used as target to test the functionality of a chimera pRNA-ribozyme. This work is important because it provides potential for the treatment of this serious infectious disease.

The hammerhead ribozyme designed by Feng et al. (Biol. Chem. 382:655-660 (2001)) cleaves a 137-nucleotide HBV-polyA substrate into two fragments of 70 and 67 nucleotides. We tested two versions of this ribozyme: RzApRNA, which contained a pRNA moiety, and RzA, which did not.

The HBV-polyA substrate RNA was generated by T7 polymerase *in vitro* transcription using a linearized plasmid as a template for run-off transcripts. This plasmid was kindly provided by laboratories led by Professor Yuan Wang and Guorong Qi in Shanghai Institute of Biochemistry, Chinese National Academy of Sciences.

A dsDNA fragment encoding the pRNA chimera, RzApRNA, was made by PCR from a linearized transcript. The sequence was inserted into a plasmid, and the RzApRNA was transcribed by T7 polymerase. This RzApRNA transcription product then underwent a cis-cleavage reaction to free itself from extraneous RNA flanking sequences. "Cis-cleavage" means a cleavage reaction where both the ribozyme and the substrate are part of the same molecule. This cis-cleavage reaction was performed by two ribozymes that flanked the chimera sequence. One cis-ribozyme was 5' to the chimera, while the other cis-ribozyme was 3' to the chimera. The cis-cleavage reaction occurred predominately during the time the RzApRNA was made. The product of the cis-cleaved transcript was the 188mer:

The cleavage reaction was performed at 37°C for 60 minutes in the presence of 20 mM Tris pH 7.5, and 20 mM MgCl₂. RzApRNA (0.539 nmol) was used to cleave HBV-polyA (0.117 nmol). Control reactions were performed by substituting water for certain RNA. The RNA for which water was substituted was omitted from the name of the control. For example, the RzApRNA control has no HBV-polyA. The samples were dialyzed against TE (10 mM Tris, 1 mM EDTA, pH 8.0) for 30 minutes on a Millipore 0.025 µm VS type membrane. 2x loading buffer (8 M urea, TBE, 0.08% bromophenol blue, 0.08% xylene cyanol) was added to the samples prior to loading them on a 15% PAGE / 8 M urea denaturing gel in TBE (0.09 M Tris-borate, 0.002 M EDTA). The gel was run at 100 volts until the xylene cyanol was 1.5 cm from the bottom of the gel. The gel was stained with ethidium bromide and visualized using EAGLE EYE II by Stratagene.

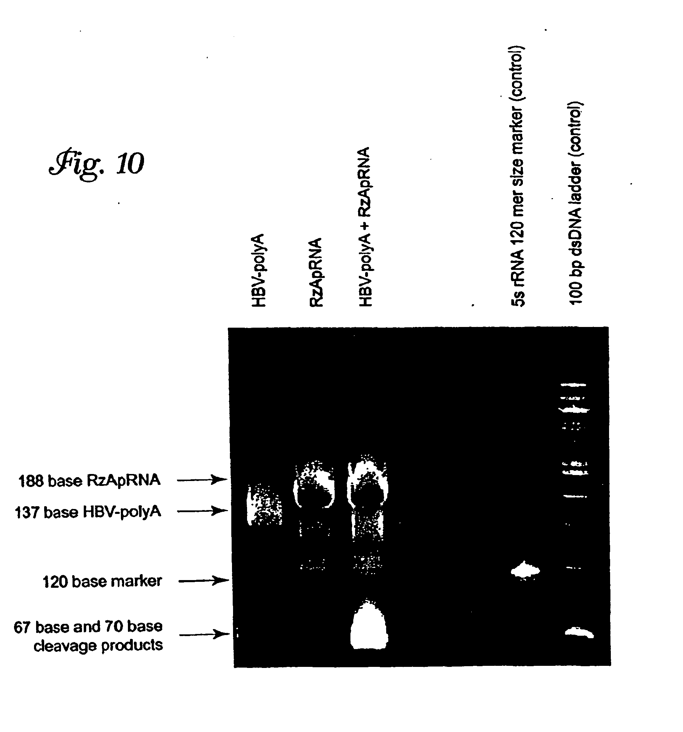

Cleavage of HBV-polyA substrate by the functional chimera RzApRNA is shown in Fig. 10. The lane labeled RzApRNA shows a control reaction that did not contain HBV-polyA, and the lane labeled HBV-polyA shows a control reaction that did not contain RzApRNA. The predicted 67 base and 70 base cleavage products are seen as one band for the cleavage reaction that included both HBV-polyA and RzApRNA.

Comparison of the cleavage efficiency of the ribozyme with and without the pRNA vector revealed a significant difference. The ribozyme RzApRNA, which contains a pRNA moiety, was able to cleave the substrate HBV-polyA with nearly100% efficiency. However, the ribozyme RzA without the pRNA moiety cleaved the substrate with an efficiency much lower than 70% (not shown).

### Example 4. In Vivo Activity of pRNA-Ribozyme Chimera against Hepatitis B Virus

A plasmid pCRzA was obtained from Professor Guorong Qi in Shanghai. This plasmid contains sequences coding for a cis-acting hammerhead ribozyme flanked by two sequences targeting Hepatitis B virus polyA signal. When this plasmid was co-transfected into HepG2 cells with the HBV genome, HBV RNA level was decreased, and hepatitis B virus replication was inhibited in a dose dependant fashion.

We constructed a plasmid pCRzApRNA substantially in accordance with Example 2. In pCRzApRNA, the hammerhead ribozyme and its flanking sequence were carried by the phi29 pRNA, generating a pRNA chimera.

Comparison of the efficiency of ribozymes with and without the pRNA moiety (RzApRNA and RzA, respectively) is in progress using the assays described in Feng et al. (Biol. Chem. 382:655-660 (2001)). Transient DNA co-transfection is being used to test the intracellular effectiveness of the chimerical ribozyme. Results of this study will be determined by Northern Blot assay. It is fully expected that the pRNA chimera RzApRNA will show enhanced performance relative to RzA in decreasing HBV RNA levels as well as inhibiting HBV replication.

### Example 5. Activity of pRNA-Ribozyme Chimera against Cancer in Cell Culture

Growth and metastasis of solid tumors requires persistent angiogenesis. Angiogenesis is a important process by which new blood vessels are formed. The protein type 12 lipoxygenase (12-LOX) in platelets makes 12-HETE (12-hydroxy-5,8,10,14-eicosatetraenoic acid) by adding O₂ to C-12 arachidonic acid. 12-LOX and its metabolites may be important factors in tumor angiogenesis. The application of this research could restrict tumor growth by preventing cancer cells from prompting blood vessels to grow in the surrounding tissue.

*In vitro* studies by Liu et al. have shown that this ribozyme, 121oxRz, efficiently cleaved the substrate (Cancer Gene Ther. 7:671-675 (2000)). Efficiency was increased when changing the reaction temperature from 37°C to 50°C. *In vivo* studies in cell culture showed that cells expressing the ribozyme from a plasmid had such a decreased level of 12-LOX mRNA that it was undetectable by Northern blotting. A control group of cells that only had a nonfunctional mutant ribozyme had only a slight decrease in the level of 12-LOX mRNA. This slight reduction in 12-LOX mRNA expression could have been the result of an antisense effect by the mutant ribozyme by merely binding to the 12-LOX mRNA without cleaving it. Cell extract was assayed for 12-LOX enzyme activity. Cells expressing ribozymes had 13% of 12-LOX enzyme activity after 6 months compared to parental cells. Cells expressing the mutant nonfunctional ribozyme had 80% of 12-LOX enzyme activity compared to parental cells (Liu et al., Cancer Gene Ther., 7:671-675, 2000). This demonstrates the activity of the ribozyme.

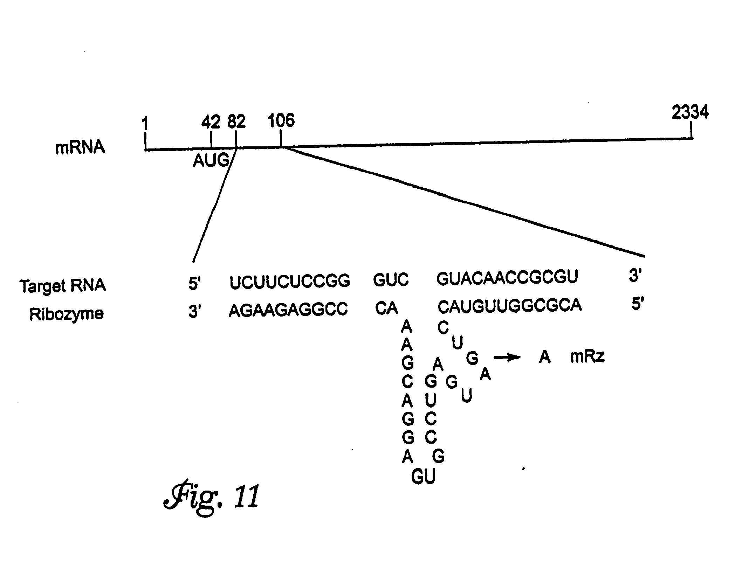

Platelet-type 12-lipoxygenase (12-lox) mRNA (Fig. 11) was selected as a target to test whether a chimera hammerhead ribozyme can function to suppress mRNA levels in human erythroleukemia (HEL) cells. We obtained the *in vitro* and *in vivo* plasmids that encode the ribozyme from Professor Tien, Director of the National Key Lab of Virus Research in which the inventor Peixuan Guo is the Advisor and Visiting Professor. The hammerhead ribozyme was inserted into our pRNA essentially using the method described in Example 2. We created the chimerical ribozyme, 121oxRzpRNA, first constructing a dsDNA template in a two step PCR reaction from oligonucleotides encoding the T7 promoter and the 121oxRz inserted into the pRNA sequence. This template was subsequently transcribed to give the 12loxRzpRNA.

Experiments to test the activity of 12loxRzpRNA will be performed. For the *in vitro* experiments, the 12loxRz and a target RNA fragment of the 12-lox mRNA (the mRNA substrate) are produced from oligonucleotides essentially using the method described in Example 2. The 12loxRz and the substrate RNA are each transcribed from their own set of two hybridized DNA oligonucleotides. One encodes the negative sense T7 polymerase promoter and the substrate sequence or the 121oxRz sequence. The other oligonucleotide encodes the positive sense T7 promoter sequence. The RNA substrate is radio-labeled using calf intestine phosphatase (CIP) and then polynucleotide kinase (PNK) with [γ³²P]-ATP.

The cleavage efficiency of two ribozymes with and without the pRNA moiety will be evaluated both *in vitro* and cells (cell culture). For the *in vitro* study, we will compare the stability of the ribozymes resistance to pH, ion concentration, RNase and cell lysate. These are factors that affect the ribozyme stability and function in the cell.

HEL cells expressing 12-lox will be used for the cell culture experiments. An empty expression cassette or the 12loxRzpRNA in an expression cassette encoding the tRNA^{val} promoter, the 12loxRzpRNA chimera, and the eukaryote polymerase III terminator sequence (5 T residues) will be delivered by transfection using electroporation. Expression of the 121oxRzpRNA chimera and 12-lox mRNA in the cells will be detected by northern blot. Nontransfected HEL cells will be used as a control. 12-LOX enzyme activity will be evaluated by the determination of whether there is a reduction in 12-HETE production in HEL cells.

For both the *in vitro* and cell culture experiments, a mutant 121oxRz and a mutant 121oxRzpRNA chimera control will be used as a second control. The mutant l2loxRz has one of its nucleotides in its conserved catalytic core domain substituted with another base, rendering the ribozyme unable to cleave the substrate RNA. The use of the non-catalytic mutant ribozymes as a second control is designed to reveal whether the native ribozyme is capable of inhibiting translation by binding to the RNA substrate (i.e., an antisense effect), as opposed to cleaving it.

### Sequence Listing Fee Text

- 1: organism name: Bacteriophage phi29/PZA
- 2: circularly permuted pRNA from bacteriophage phi29
- 3: RNA chimera containing phi29 pRNA and hammerhead ribozyme
- 4: U7snRNA substrate
- 5: anti-12-Lox ribozyme
- 6: substrate RNA
- 7: pRNA chimera
- 8: linking loop
- 9: U7 substrate
- 10: Hammerhead ribozyme
- 11: organism name: Bacteriophage SF5'
- 12: organism name: Bacteriophage B103
- 13: (not used)
- 14: organism name: Bacteriophage M2/NF
- 15: organism name: Bacteriophage GA1
- 16: aptpRNA
- 17: pRzApRNA

## Claims

1. A pRNA chimera comprising:
(a) a pRNA region; and
(b) a spacer region comprising a biologically active RNA, the spacer region covalently linked at its 5' and 3' ends to the pRNA region;
wherein the pRNA region comprises:
(i) in the 5' to 3' direction beginning at the covalent linkage of the pRNA with the 3' end of the spacer region
a first loop (22);
a second loop (24); and
a lower stem-loop structure comprising a bulge (25), a first stem section (26) and a third loop (27);
(ii) a second stem section (20) interposed between the spacer region and the stem-loop structure;
(iii) a third stem section (21) interposed between the stem-loop structure and the first loop (22);
(iv) a fourth stem section (23) interposed between the first loop (22) and the second loop (24); and
(v) an opening defining 5' and 3' ends of the pRNA chimera.

2. The pRNA chimera of claim 1 wherein the pRNA region comprises a circularly permuted pRNA of a bacteriophage selected from the group consisting of bacteriophage ϕ29, SF5', B103, PZA, M2, NF and GA1.

3. The pRNA chimera of claim 1 or 2 where the biologically active RNA is selected from the group consisting of a ribozyme and an antisense RNA.

4. The pRNA chimera of any one of claims 1 to 3 wherein the spacer region comprises a biologically active RNA comprising a ribozyme and first and second nucleotide strings interposed between the ribozyme and the pRNA region.

5. A DNA molecule comprising a nucleotide sequence that encodes a pRNA chimera according to any one of claims 1 to 4.

6. An *in vitro* method for making a pRNA chimera comprising transcribing the DNA of claim 5 to yield the pRNA chimera.

7. A method for making a pRNA chimera comprising transcribing the DNA of claim 5 in a non-animal cell to yield the pRNA chimera.

8. The method of claim 6 or 7 wherein transcription occurs in a cell present in cell culture or in a cell explanted from an organism.

9. The method of claim 7 wherein transcription occurs in a cell present in an organism.

10. The method of claim 8 wherein transcription occurs *in vitro,* the method further comprises using polymerase chain reaction on a DNA template to generate the DNA. encoding the pRNA chimera.

11. The method of any one of claims 6 to 9 further comprising generating the DNA encoding the pRNA chimera by cloning the DNA into a plasmid and replicating the plasmid.

12. A method for determining whether an RNA molecule interacts with a test molecule, the method comprising:
providing a pRNA chimera according to anyone of claims 1 to 4, comprising a pRNA region flanking a spacer region comprising the RNA molecule;
immobilizing the pRNA chimera on a substrate;
contacting the immobilized pRNA chimera with a test molecule; and
detecting whether the test molecule interacts with the RNA molecule.

13. An *in vitro* method for delivering a biologically active RNA to a cell comprising introducing a pRNA chimera according to any of claims 1 to 4 into the cell.

14. A method for delivering a biologically active RNA to a non-animal cell comprising introducing a pRNA chimera according to any of claims 1 to 4 into the cell.

15. The method of claim 14 wherein the cell is present in an organism.

16. The method of any of claims 13 to 15 wherein introducing the pRNA chimera into the cell comprises:
transfecting the cell with a DNA molecule comprising a nucleotide sequence that operably encodes the pRNA chimera and
causing transcription of the DNA molecule in the cell to yield the biologically active RNA.

17. The method of any of claims 13 to 15 wherein the pRNA chimera is transfected directly into the cell.

18. The method of any of claims 13, 14, 16 and 17 wherein the cell is present in cell culture, or is explanted from an organism.

19. The method of any of claims 13 to 18 wherein the cell is a plant cell or claims 13 or 16 to 18 wherein the cell is an animal cell.

20. The pRNA chimera of any one of claims 1 to 4 for use in therapy or for use in delivering a biologically active RNA to a cell.

21. The DNA molecule of claim 5 for use in therapy or for use in making a pRNA chimera as defined in any one of claims 1 to 4.

22. Use of the pRNA chimera of any one of claims 1 to 4 in the manufacture of a medicament for the treatment of cancer or pathogen infection.

23. Use of a DNA molecule of claim 5 in the manufacture of a medicament for the treatment of cancer or pathogen infection.

24. Pharmaceutical composition comprising the pRNA chimera of any one of claims 1 to 4 for delivery of a biologically active RNA to a cell.

25. Pharmaceutical composition comprising the DNA molecule of claim 5 for use in making a pRNA chimera as defined in any one of claims 1 to 4."

## Patentansprüche

1. pRNA-Chimäre, umfassend:
(a) einen pRNA-Bereich; und
(b) einen Spacer-Bereich, der eine biologisch aktive RNA umfasst, wobei der Spacer-Bereich an seinen 5'- und 3'-Enden mit der pRNA kovalent verbunden ist;
wobei der pRNA-Bereich umfasst:
(i) in der 5'- zu 3'-Richtung, beginnend bei der kovalenten Verbindung der pRNA mit dem 3'-Ende des Spacer-Bereichs
eine erste Schleife (22);
eine zweite Schleife (24); und
eine untere Haarnadelstruktur, die eine Ausbuchtung (25), einen ersten Stammbereich (26) und eine dritte Schleife (27) umfasst;
(ii) einen zweiten Stammbereich (20) der zwischen dem Spacer-Bereich und der Haarnadelstruktur eingefügt ist;
(iii) einen dritten Stammbereich (21), der zwischen der Haarnadelstruktur und der ersten Schleife (22) eingefügt ist;
(iv) einen vierten Stammbereich (23), der zwischen der ersten Schleife (22) und der zweiten Schleife (24) eingefügt ist; und
(v) eine Öffnung, die die 5'- und 3'-Enden der pRNA-Chimäre definiert.

2. pRNA-Chimäre nach Anspruch 1, wobei der pRNA-Bereich eine kreisförmig permutierte pRNA eines Bacteriophagen, ausgewählt aus der Gruppe bestehend aus Bacteriophage ϕ29, SF5', B103, PZA, M2, NF und GA1, umfasst.

3. pRNA-Chimäre nach Anspruch 1 oder 2, wobei die biologisch aktive RNA ausgewählt ist aus der Gruppe bestehend aus einem Ribozym und einer Antisense-RNA.

4. pRNA-Chimäre nach einem der Ansprüche 1 bis 3, wobei der Spacer-Bereich eine biologisch aktive RNA umfasst, die ein Ribozym und erste und zweite Nucleotidstränge umfasst, welche zwischen dem Ribozym und dem pRNA-Bereich eingefügt sind.

5. DNA-Molekül, umfassend eine Nucleotidsequenz, die eine pRNA-Chimäre nach einem der Ansprüche 1 bis 4 codiert.

6. In vitro-Verfahren für die Herstellung einer pRNA-Chimäre, umfassend das Transkribieren der DNA nach Anspruch 5 für die Erstellung der pRNA-Chimäre.

7. Verfahren für die Herstellung einer pRNA-Chimäre, umfassend das Transkribieren der DNA nach Anspruch 5 in einer nicht-tierischen Zelle für die Erstellung einer pRNA-Chimäre.

8. Verfahren nach Anspruch 6 oder 7, wobei die Transkription in einer Zelle stattfindet, die in Zellkultur vorhanden ist, oder in einer Zelle, die aus einem Organismus explantiert wurde.

9. Verfahren nach Anspruch 7, wobei die Transkription in einer Zelle stattfindet, die in einem Organismus vorhanden ist.

10. Verfahren nach Anspruch 8, wobei die Transkription *in vitro* stattfindet, wobei das Verfahren ferner die Verwendung der Polymerasen-Kettenreaktion an einer DNA-Matrize umfasst, zur Erzeugung der DNA, welche die pRNA-Chimäre codiert.

11. Verfahren nach einem der Ansprüche 6 bis 9, ferner umfassend die Erzeugung der DNA, die die pRNA-Chimäre codiert, durch das Clonieren der DNA in ein Plasmid und das Replizieren des Plasmids.

12. Verfahren zur Bestimmung, ob ein RNA-Molekül mit einem Testmolekül interagiert, wobei das Verfahren umfasst:
das Bereitstellen einer pRNA-Chimäre nach einem der Ansprüche 1 bis 4, umfassend einen pRNA-Bereich, der einen Spacer-Bereich flankiert, der das RNA-Molekül umfasst;
das Immobilisieren der pRNA-Chimäre auf einem Substrat;
das Inkontaktbringen der immobilisierten pRNA-Chimäre mit einem Testmolekül; und
das Bestimmen, ob das Testmolekül mit dem RNA-Molekül interagiert.

13. In vitro-Verfahren zur Überführung einer biologisch aktiven RNA in eine Zelle, umfassend das Einführen einer pRNA-Chimäre nach einem der Ansprüche 1 bis 4 in die Zelle.

14. Verfahren zur Überführung einer biologisch aktiven RNA in eine nicht-tierische Zelle, umfassend das Einführen einer pRNA-Chimäre nach einem der Ansprüche 1 bis 4 in die Zelle.

15. Verfahren nach Anspruch 14, wobei die Zelle in einem Organismus vorhanden ist.

16. Verfahren nach einem der Ansprüche 13 bis 15, wobei das Einführen der pRNA-Chimäre in der Zelle umfasst:
das Transfizieren der Zelle mit einem DNA-Molekül, umfassend eine Nucleotidsequenz, die die pRNA-Chimäre funktionell codiert, und
das Auslösen der Transkription des DNA-Moleküls in der Zelle für die Erstellung der biologisch aktiven RNA.

17. Verfahren nach einem der Ansprüche 13 bis 15, wobei die pRNA-Chimäre direkt in die Zelle transfiziert wird.

18. Verfahren nach einem der Ansprüche 13, 14, 16 und 17, wobei die Zelle in einer Zellkultur vorhanden ist oder aus einem Organismus explantiert wurde.

19. Verfahren nach einem der Ansprüche 13 bis 18, wobei die Zelle eine Pflanzenzelle ist, oder nach einem der Ansprüche 13 oder 16 bis 18, wobei die Zelle eine tierische Zelle ist.

20. pRNA-Chimäre nach einem der Ansprüche 1 bis 4 für die Verwendung in der Therapie oder für die Verwendung zum Überführen einer biologisch aktiven RNA in eine Zelle.

21. DNA-Molekül nach Anspruch 5 für die Verwendung in der Therapie oder für die Verwendung zur Herstellung einer pRNA-Chimäre nach einem der Ansprüche 1 bis 4.

22. Verwendung der pRNA-Chimäre nach einem der Ansprüche 1 bis 4 für die Herstellung eines Arzneimittels zur Behandlung von Krebs oder einer Infektion mit einem Krankheitserreger.

23. Verwendung eines DNA-Moleküls nach Anspruch 5 für die Herstellung eines Arzneimittels zur Behandlung von Krebs oder einer Infektion mit einem Krankheitserreger.

24. Arzneimittel, umfassend die pRNA-Chimäre nach einem der Ansprüche 1 bis 4 zur Überführung einer biologisch aktiven RNA in eine Zelle.

25. Arzneimittel, umfassend das DNA-Molekül nach Anspruch 5 für die Verwendung zur Herstellung einer pRNA-Chimäre nach einem der Ansprüche 1 bis 4.

## Revendications

1. Chimère d'ARNp comprenant:
(a) une région d'ARNp ; et
(b) une région espaceur comprenant un ARN biologiquement actif, la région espaceur étant liée de façon covalente à ses extrémités 5' et 3' à la région d'ARNp ;
dans laquelle la région d'ARNp comprend :
(i) dans la direction de 5' vers 3', en commençant à la liaison covalente de l'ARNp avec l'extrémité 3' de la région espaceur,
une première boucle (22) ;
une deuxième boucle (24) ; et
une structure tige-boucle inférieure comprenant un renflement (25), une première section de tige (26) et une troisième boucle (27) ;
(ii) une deuxième section de tige (20) interposée entre la région espaceur et la structure tige-boucle ;
(iii) une troisième section de tige (21) interposée entre la structure tige-boucle et la première boucle (22) ;
(iv) une quatrième section de tige (23) interposée entre la première boucle (22) et la deuxième boucle (24) ; et
(v) une ouverture définissant les extrémités 5' et 3' de la chimère d'ARNp.

2. Chimère d'ARNp selon la revendication 1, dans laquelle la région d'ARNp comprend un ARNp, permuté de manière circulaire, d'un bactériophage choisi dans le groupe constitué par les bactériophages ϕ29, SF5', B103, PZA, M2, NF et GA1.

3. Chimère d'ARNp selon la revendication 1 ou 2, dans laquelle l'ARN biologiquement actif est choisi dans le groupe constitué par un ribozyme et un ARN antisens.

4. Chimère d'ARNp selon l'une quelconque des revendications 1 à 3, dans laquelle la région espaceur comprend un ARN biologiquement actif comprenant un ribozyme et des première et deuxième chaînes nucléotidiques interposées entre le ribozyme et la région d'ARNp.

5. Molécule d'ADN comprenant une séquence de nucléotides qui code une chimère d'ARNp selon l'une quelconque des revendications 1 à 4.

6. Procédé in vitro pour produire une chimère d'ARNp, comprenant la transcription de l'ADN de la revendication 5 pour engendrer la chimère d'ARNp.

7. Procédé pour produire une chimère d'ARNp, comprenant la transcription de l'ADN de la revendication 5 dans une cellule non animale pour engendrer la chimère d'ARNp.

8. Procédé selon la revendication 6 ou 7, dans lequel la transcription a lieu dans une cellule présente dans une culture cellulaire ou dans une cellule explantée hors d'un organisme.

9. Procédé selon la revendication 7, dans lequel la transcription a lieu dans une cellule présente dans un organisme.

10. Procédé selon la revendication 8, dans lequel la transcription a lieu in vitro, le procédé comprenant en outre l'utilisation d'une amplification en chaîne par polymérase sur une matrice d'ADN pour générer l'ADN codant la chimère d'ARNp.

11. Procédé selon l'une quelconque des revendications 6 à 9, comprenant en outre la génération de l'ADN codant la chimère d'ARNp par clonage de l'ADN dans un plasmide et réplication du plasmide.

12. Procédé pour déterminer si une molécule d'ARN interagit avec une molécule de test, le procédé comprenant :
l'obtention d'une chimère d'ARNp selon l'une quelconque des revendications 1 à 4, comprenant une région d'ARNp flanquant une région espaceur comprenant la molécule d'ARN ;
l'immobilisation de la chimère d'ARNp sur un substrat ;
la mise en contact de la chimère d'ARNp immobilisée avec une molécule de test ; et
la détection du fait que la molécule de test interagit ou non avec la molécule d'ARN.

13. Procédé in vitro pour délivrer un ARN biologiquement actif à une cellule, comprenant l'introduction dans la cellule d'une chimère d'ARNp selon l'une quelconque des revendications 1 à 4.

14. Procédé pour délivrer un ARN biologiquement actif à une cellule non animale, comprenant l'introduction dans la cellule d'une chimère d'ARNp selon l'une quelconque des revendications 1 à 4.

15. Procédé selon la revendication 14, dans lequel la cellule est présente dans un organisme.

16. Procédé selon l'une quelconque des revendications 13 à 15, dans lequel l'introduction de la chimère d'ARNp dans la cellule comprend :
la transfection de la cellule avec une molécule d'ADN comprenant une séquence de nucléotides qui code de manière opérable la chimère d'ARNp, et
le déclenchement de la transcription de la molécule d'ADN dans la cellule pour engendrer l'ADN biologiquement actif.

17. Procédé selon l'une quelconque des revendications 13 à 15, dans lequel la chimère d'ARNp est transfectée directement dans la cellule.

18. Procédé selon l'une quelconque des revendications 13, 14, 16 et 17, dans lequel la cellule est présente dans une culture cellulaire, ou est explantée hors d'un organisme.

19. Procédé selon l'une quelconque des revendications 13 à 18, dans lequel la cellule est une cellule végétale, ou selon les revendications 13 ou 16 à 18, dans lequel la cellule est une cellule animale.

20. Chimère d'ARNp selon l'une quelconque des revendications 1 à 4, pour une utilisation en thérapie ou pour une utilisation dans la délivrance d'un ARN biologiquement actif à une cellule.

21. Molécule d'ADN selon la revendication 5, pour une utilisation en thérapie ou pour une utilisation dans la préparation d'une chimère d'ARNp telle que définie dans l'une quelconque des revendications 1 à 4.

22. Utilisation de la chimère d'ARNp de l'une quelconque des revendications 1 à 4 dans la fabrication d'un médicament pour le traitement d'un cancer ou d'une infection par un pathogène.

23. Utilisation d'une molécule d'ADN de la revendication 5 dans la fabrication d'un médicament pour le traitement d'un cancer ou d'une infection par un pathogène.

24. Composition pharmaceutique comprenant la chimère d'ARNp de l'une quelconque des revendications 1 à 4 pour la délivrance à une cellule d'un ARN biologiquement actif.

25. Composition pharmaceutique comprenant la molécule d'ADN de la revendication 5 pour une utilisation dans la préparation d'une chimère d'ARNp telle que définie dans l'une quelconque des revendications 1 à 4.
